**Europäisches Patentamt**

**European Patent Office**

**Office européen des brevets**

(19)

(11) Numéro de publication: **0 154 758**
**B1**

(12)

# FASCICULE DE BREVET EUROPEEN

(45) Date de publication du fascicule du brevet:
**07.10.87**

(51) Int. Cl.⁴: **A 61 F 5/01,** A 61 F 13/06

(21) Numéro de dépôt: **84420042.8**

(22) Date de dépôt: **09.03.84**

---

(54) **Genouillère de réadaptation fonctionnelle.**

---

(43) Date de publication de la demande:
**18.09.85 Bulletin 85/38**

(45) Mention de la délivrance du brevet:
**07.10.87 Bulletin 87/41**

(84) Etats contractants désignés:
**AT BE CH DE FR GB IT LI LU NL SE**

(56) Documents cités:
**EP - A - 0 067 319**
**CH - A - 397 150**
**FR - A - 2 532 839**
**US - A - 4 287 885**
**US - A - 4 366 813**

(73) Titulaire: **Tricotage Elastique du Forez, S.A., 219 route d'Andrézieux, F-42170 Saint-Just-Saint-Rambert (FR)**

(72) Inventeur: **Bertheas, Michel, 1, rue Jean-Baptiste Marcet, F-42170 Saint-Just-Saint-Rambert (FR)**

(74) Mandataire: **Dupuis, François et al, Cabinet Charras 3 Place de l'Hôtel-de-Ville, F-42000 St.Etienne (FR)**

---

## Description

L'invention est relative à une genouillère de réadaptation fonctionelle.

La plupart des genouillères sont constituées par une enveloppe en tissu élastique apte à envelopper la jambe de part et d'autre du genou pour assurer le maintien de cette articulation après un effort violent ou un choc ayant détérioré ou distendu les ligaments, ou après une opération.

D'autres, comme décrit dans le Brevet Européen 0 067 319, comportent une ouverture antérieure pour le passage de la rotule, éventuellement bordée intérieurement par un coussin annulaire et des tiges de renfort garantissant une position de flexion déterminée.

De par leur structure, de telles genouillères ne peuvent qu'assurer un rôle de maintien à l'exclusion de toute réadaptation fonctionnelle de l'articulation.

Il est cité au titre de l'art antérieur, les Brevets US 4 366 813 et 4 287 885. Concernant plus particulièrement le Brevet US 4 287 885, celui décrit une genouillère élastique avec une ouverture centrale autour de la rotule avec l'utilisation d'une feuille intermédiaire rigide entre la structure textile de la genouillère et le coussinet s'adaptant sur la périphérie de ladite ouverture et assurant la contention de la rotule. La feuille intermédiaire indéformable lors du mouvement du genou stabilise en position le coussinet. Ce coussinet, dans une variante de réalisation, présente une forme en fer à cheval dont l'ouverture encadre le logement rotulien sans cependant assurer une fonction de contention du ligament rotulien.

La présente invention a pour but de fournir une genouillère qui, outre le rôle contentif classique à toute genouillère élastique, soulage les contraintes mécaniques imposées à l'appareil rotulien lors des mouvements en charge (courses, sauts, marche).

Cette genouillère de réadaptation fonctionnelle est du type composé de:

— une enveloppe tubulaire élastique transversalement,

— fourreaux solidaires de l'enveloppe présentant chacun une partie supérieure et une partie inférieure qui forment entre elles et au repos une angulation dont le sommet est sensiblement dans l'axe transversal d'un évidement rotulien,

— moyens élastiques latéraux constitués par des ressorts spiralés plats disposés dans les fourreaux,

— moyens de serrage sur la jambe,

caractérisé en ce que la genouillère comprend une ouverture frontale pour le passage de la rotule, ouverture bordée intérieurement par un coussin, ledit coussin présentant une fente inférieure de contention du ligament rotulien, et en ce que l'angle entre les deux parties de chaque fourreau est compris entre 163° et 168°.

L'ouverture antérieure de la genouillère et la fente inférieure du coussin assurent une excellente contention périrotulienne. Cet ensemble s'adapte parfaitement aux reliefs périrotuliens sans risque de striction traumatisante avec autant de confort ed d'efficacité dans la position de repos debout, que dans le mouvement.

L'angulation, qui demeure l'effet de contrainte imposé par toute arthrose, à été choisie en fonction de la position de repos que prend spontanément le genou chez l'homme debout (position alternée, position de repos militaire). D'autre part, la position en flexum est aussi spontanément prise par tout genou traumatisé qui souffre.

Cette genouillère a donc l'originalité de ne pas s'opposer au flexum nécessaire au repos ou à l'attitude antialgique de cette articulation.

De plus, cette angulation est réductible en exerçant une force très faible ce qui lui permet de s'adapter à l'extension totale du genou lors des exercices de courses ou de sauts et, en particulier, lors des exercices de rééducation fonctionnelle.

Il est à noter que la seule forme particulière des fourreaux, facilement réalisables par des coutures, assure le positionnement des ressorts et permet à ces derniers de contribuer à la réadaptation fonctionnelle du genou.

Avantageusement, l'angulation de 165° et le sommet de l'angle formé par cette angulation est sensiblement dans l'axe transversal de l'évidement rotulien.

D'autres caractéristiques et avantages ressortiront de la description qui suit en référence au dessin schématique annexé représentant, à titre d'exemple non limitatif, une forme d'exécution de cette genouillère.

Figure 1 est une vue de côté en élévation de la genouillère la montrant lorsqu'elle est positionnée sur un genou,

Figure 2 en est une vue de côté en élévation la montrant, à échelle agrandie, au repos,

Figure 3 est une vue partielle de face en élévation montrant, à échelle agrandie, la structure du ressort utilisé.

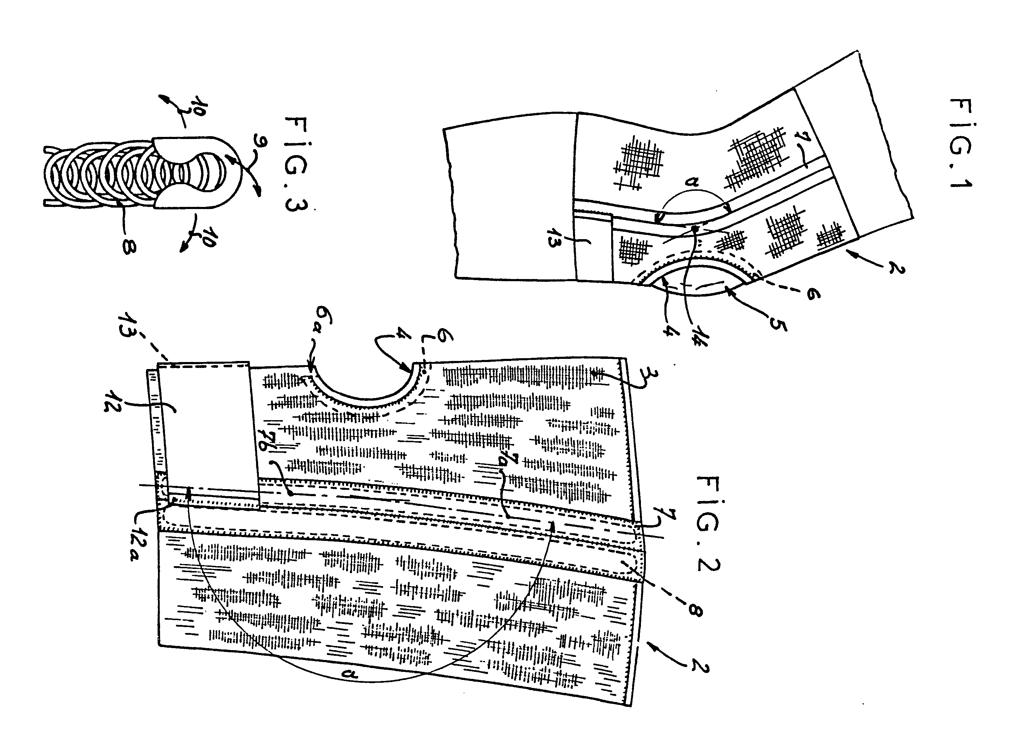

Cette genouillère, désignée de façon générale par (2), est composée, de façon connue, par une enveloppe tubulaire (3) en tissu, élastique au moins dans le sens transversal. Cette enveloppe a une forme générale tronconique, allant en s'évasant de bas en haut. Elle comporte, dans sa partie frontale, une ouverture antérieure (4) pour le passage de la rotule (5) du genou. Cette ouverture est, de façon connue, bordée intérieurement par un coussin annulaire (6) représenté en traits interrompus aux figures 1 et 2. Ce coussin est interrompu dans sa partie inférieure pour former une fente inférieure (6a) sous le tissu de la genouillère, fente permettant le passage du ligament rotulien.

Cette genouillère comporte, latéralement de chaque côté, au moins un et, de préférence, deux fourreaux (7) dans chacun desquels est disposé un ressort spiralé et plat, du type représenté en (8) à la figure 3. Ce ressort, qui est en général utilisé dans les gaines et autres éléments de contention, présente une grande flexibilité d'avant en arrière, comme représenté par la flèche (9), mais aussi latéralement comme représenté par les flèches (10) à la figure 3. Chacun des foureaux, qui est continu, est en fait composé d'une partie supérieure (7a) et inférieure (7b) formant entre elles un angle (a) compris entre 163° et 168° et, de préférence, de l'ordre de 165°. Dans cet agencement, lorsque la genouillère est au repos, les paires de ressort latérales sont immobilisées dans cette position par les fourreaux, comme montré à la figure 2.

A sa partie inférieure, la genouillère est munie de moyens de serrage qui sont constitués par deux bandes (12) et (13), dont l'une des extrémités est libre, et dont l'autre extrémité, par exemple celle (12a) à la figure 12, est fixée sur la genouillère au niveau des fourreaux latéraux correspondants. Ces deux bandes de serrage sont munies de moyens d'accrochage complémentaires.

Lorsque la genouillère est passée sur le genou, comme montré à la figure 1, le sommet (14) de l'angle (a) formé par les ressorts (8) et fourreaux (7) est sensiblement dans l'axe transversal de la cavité rotulienne du genou (5).

Une telle genouillère peut être utilisée pour supprimer la douleur ou permettre la pratique sportive malgré des chondrites rotuliennes douloureuses associées ou non à une surpression rotulienne externe ou à une dysplasie, pour soulager des cas d'instabilité rotulienne, des tendinites d'insertion rotulienne du tendon rotulien ou après une transposition chirurgicale de la tubérosité tibiale. Dans les cas hyperalgiques, la genouillère peut être portée dans la journée pour diminuer la douleur de la marche et pour la pratique des sports. Son utilisation la plus intéressante est de permettre la pratique sportive, y compris les sports nautiques, en modifiant les contraintes mécaniques subies par l'appareil rotulien. Dans les cas post-opératoires, la réaxation rotulienne qu'elle assure est intéressante, car elle permet une reprise du footing et des exercices de réadaptation sportive sur le terrain plus facilement et plus précocement. Les qualités musculaires et la proprioception s'en trouvent ainsi plus vite et plus complètement récupérés.

De manière générale, cette genouillère donne des résultats fonctionnels et subjectifs remarquables dans les cas de souffrance rotulienne et para-rotulienne. Elle favorise la pratique sportive et constitue, bien souvent, le seul moyen de continuer cette pratique. Après réaxation rotulienne chirurgicale, elle accélère et améliore la réadaptation sportive. Elle rend des services intéressants en cas de souffrance du ligament rotulien et donne des résultats spéctaculaires dans les chondrites rotuliennes et les subluxations rotuliennes.

## Revendication

Genouillère de réadaptation fonctionnelle du type composé de:
— une enveloppe (3) tubulaire élastique transversalement,
— fourreaux (7) solidaires de l'enveloppe (3) présentant chacun une partie supérieure (7a) et une partie inférieure (7b) qui forment entre elles et au repos une angulation dont le sommet est sensiblement dans l'axe transversal d'un évidement rotulien,
— moyens élastiques latéraux constitués par des ressorts spiralés plats (8) disposés dans les fourreaux (7),
— moyens de serrage (12, 13) sur la jambe, caractérisé en ce que la genouillère comprend une ouverture frontale pour le passage de la rotule, ouverture bordée intérieurement par un coussin (6), ledit coussin présentant une fente inférieure (6a) de contention du ligament rotulien, et en ce que l'angle entre les deux parties (7a, 7b) de chaque fourreau (7) est compris entre 163° et 168°.

## Patentanspruch

Funktionelle Umerziehungskniebandage in der folgenden Ausführung:
— eine rohrförmige, in Querrichtung elastische Umhüllung (3);
— mit der Umhüllung (3) festgemachte Scheiden (7) die jede eine einen Oberteil (7a) und einen Unterteil (7b) aufweisen, die zwischeneinander und in der Ruhestellung eine Winkelformgebung bilden, deren Scheitelpunkt etwa in der Querachse einer Kniescheibenaussparung liegt;
— in den Scheiden (7) angebrachte, aus flachen Schraubenfedern (8) seitliche elastische Mittel;
— Klemmenmittel (12, 13) zum Festbinden auf dem Bein,
dadurch gekennzeichnet, dass die Kniebandage eine durch ein Kissen (6) innenseitig gefütterte Vorderöffnung zum Durchlassen der Kniescheibe aufweist, wobei das besagte Kissen mit einem Niederschlitz (6a) zum Aufhalten des Kniescheibemuskelbands versehen ist, und dass der Winkel zwischen den beiden Teilen (7a, 7b) jeder Scheibe (7) zwischen 163° und 168° liegt.

## Claim

Functional readaptation knee-pad of the type consisting of:
— a transversely elastic tubular casing (3),
— sheathings (7) integral with the casing (3) having each one an upper portion (7a) and a lower portion (7b) which form therebetween and at rest an angular shape the apex of which is appreciably within the transverse axis of a knee-cap recess,
— lateral elastic means formed by flat spiral springs (8) arranged within the sheathings (7),
— tightening means (12, 13) on the leg, characterised in that the knee-pad includes a front opening for the passage of the knee-cap, said opening being lined internally by a cushion (6), said cushion being provided with a lower slot (6a) for retaining the patellar ligament, and in that the angle between the two portions (7a, 7b) of each sheathing (7) is comprised between 163° and 168°.

# FİG.1

# FİG.2

# FİG.3